# EUROPEAN PATENT APPLICATION

(11) **EP 1 816 479 A1**
(43) Date of publication of application: **08.08.2007**
(21) Application number: 05809479.8
(22) Date of filing: 21.11.2005
(51) Int. Cl.: G01N 35/00, G01N 1/36, G01N 37/00

(54) **SENSOR DEVICE**

(30) Priority: 25.11.2004 JP 2004340746
(71) Applicant: MATSUSHITA ELECTRIC INDUSTRIAL CO., LTD., Osaka 571-8501 (JP)
(72) Inventor: NAKAMINAMI, Takahiro, Matsushita Electric Industrial Co., Ltd., Chuo-ku Osaka-shi Osaka 540-6319 (JP); KAMEI, Akihito Matsushita Electric Industrial Co., Ltd., Chuo-ku Osaka-shi Osaka 540-6319 (JP)
(74) Representative: TBK-Patent
(86) International application number: PCT/JP2005/021380
(87) International publication number: WO 2006/057225

(57) **Abstract**

A sensor device enabling quick and uniform dissolution of reagents is provided. The sensor device includes a holding member having a liquid sample holding section for holding a liquid sample containing an analyte. A reagent that reacts or interacts with the analyte in the liquid sample and an effervescent agent that effervesces upon contact with the liquid sample are held at a position of the holding member where they are to come into contact with the liquid sample introduced into the liquid sample holding section.

## Description

### Technical Field

The present invention relates to a sensor device that quantifies an analyte in a liquid sample, particularly a biological sample such as blood or urine.

### Background Art

In recent years, various types of sensor devices utilizing specific reactions of enzymes or the like, or specific interactions of antibodies or the like have been developed. An enzyme catalytically reacts with an analyte to produce a compound that is different from the analyte. An antibody interacts with an analyte and combines with the analyte to produce a complex.
A method of glucose quantification is described below as an example of methods for quantifying a substrate (analyte) in a liquid sample. A commonly known electrochemical method of glucose quantification is a method utilizing glucose oxidase (EC1.1.3.4: hereinafter referred to as GOD), an electron mediator, and electrodes.

GOD selectively oxidizes β-D-glucose, which is a substrate, and converts it to D-glucono-δ-lactone. In this oxidation reaction process by GOD, if the reaction solution contains a suitable electron mediator, for example, potassium ferricyanide, a metal complex such as a ferrocene derivative, or an organic compound such as a quinone derivative, the oxidized electron mediator is reduced by electrons derived from glucose. The amount of the reduced electron mediator produced and the production speed thereof are dependent on the glucose concentration in the liquid sample. Therefore, glucose quantification can be made by oxidizing the reduced electron mediator at an electrode, measuring the oxidation current, and obtaining the amount of the oxidized electron mediator produced or the production speed thereof.

A known example of glucose sensors enabling simple quantification of glucose is a sensor device in which a known amount of GOD and such an electron mediator are carried in a stable state on an electrode (e.g., see Patent Document 1). This biosensor is detachably connected to a measuring device and capable of readily determining glucose concentration by simply introducing a liquid sample to the reagent holding section of the sensor. Such an approach is applicable to not only glucose quantification but also quantification of other substrates or compounds contained in a liquid sample.

For example, the quantification of an antigen contained in a liquid sample is performed by using an antibody with high selective binding affinity for the antigen. Among such measurement methods, a method of detecting agglutination complexes formed by an antigen-antibody reaction is well known. Exemplary measurement methods include nephelometry, turbidimetry, and slide agglutination. These measurement methods are collectively called homogeneous immunoreaction assays, since an antibody and its antigen are homogeneously dispersed in a solution upon quantification (e.g., see Patent Document 2).

In this case, the reaction between the antigen and the antibody results in the formation of agglutination complexes, so that the reaction system becomes turbid. The turbidity depends on the amount of antigen and antibody.
Nephelometry and turbidimetry are optical measurement methods utilizing the turbidity. Specifically, nephelometry is a measurement method based on the amount of light scattered by a reaction system, and turbidimetry is a measurement method based on the amount of decrease in transmitted light caused by scattering in a reaction system. Generally, these methods can measure the same reaction system as a measuring object, and an object that can be measured by one of these methods can also be measured by the other method.

Slide agglutination is a method of visually checking the turbidity caused by the formation of agglutination complexes on a slide glass or the like, and the same reaction system as that measured by nephelometry and turbidimetry can be used.
Immunoagglutination assay is a kind of immunoassay. When the binding reaction of an antibody to the antigen proceeds, cross-linked antigen-antibody complexes and aggregates thereof are produced. As a result, relatively large-sized particles are present in the liquid sample. According to this method, it is believed that antigen quantification can be made by using a sensor that carries a plurality of kinds of antibodies in its liquid sample holding section. For example, by measuring the intensity of scattered light having a specific wavelength in a reaction solution, the concentration of an antigen correlated therewith and contained in the liquid sample can be estimated.

The above-described sensors are equipped with a disposable sensor body. In this case, a reagent layer, composed mainly of a combination of an enzyme and an electron mediator or a combination of an antibody and a water-soluble polymer, is carried in an almost dry state on the liquid sample holding section. Thus, the dissolution of the reagents by a liquid sample is slow, thereby causing a problem of long measurement/quantification time.
Also, when making a series of measurements, measurement environments, such as how each reagent is introduced and temperature, are not necessarily the same, and thus the dissolution of reagents differs among the respective measurements. Therefore, even when the analyte concentration is the same, quantification results may vary, which is a problem.

In order to solve these problems, for example, Patent Document 3 proposes a measuring device whose sensor and sensor holder serve as a stirrer for stirring a liquid sample in a cell, and Patent Document 4 proposes a semiconductor biosensor capable of vibration.
Patent Document 1: Japanese Laid-Open Patent Publication No. Hei 03-202764
Patent Document 2: Japanese Laid-Open Patent Publication No. 2003-66047
Patent Document 3: Japanese Laid-Open Patent Publication No. Sho 62-145159
Patent Document 4: Japanese Laid-Open Patent Publication No. Hei 1-206254

### Disclosure of Invention

### Problem That the Invention Is to Solve

However, the sensors disclosed in Patent Documents 3 and 4 need a driving mechanism, such as a motor or a piezoelectric actuator for rotating the stirrer or vibrating the sensor, thereby making the apparatus complicated and larger in size.
In view of the above conventional problems, it is therefore an object of the present invention to provide a sensor device that enables quick and uniform dissolution of reagents, has a simple structure, and is capable of size reduction.

### Means for Solving the Problem

In order to solve the above problems, a sensor device of the present invention includes a holding member having a liquid sample holding section for holding a liquid sample containing an analyte. A reagent that reacts or interacts with the analyte in the liquid sample and an effervescent agent that effervesces upon contact with the liquid sample are held at a position of the holding member where the liquid sample and the effervescent agent are to come into contact with the liquid sample introduced into the liquid sample holding section.
The effervescent agent preferably effervesces by producing carbon dioxide.
The effervescent agent preferably includes at least a hydrogen carbonate or a carbonate.
Preferably, an organic acid is further held at the above-mentioned position of the holding member.
The organic acid is preferably at least one selected from the group consisting of maleic acid, maleic anhydride, succinic acid, succinic anhydride, phthalic acid, phthalic anhydride, citric acid, fumaric acid, and tartaric acid.
The liquid sample is preferably a liquid derived from a living body.
The reagent is preferably an enzyme or an antibody.
At least a part of the surface of the liquid sample holding section is preferably subjected to a hydrophilic treatment.

It is preferably an apparatus or method for measuring an analyte in a liquid sample, using the above-mentioned sensor device.
That is, the present invention provides a measuring apparatus including: a sensor holder for holding the sensor device; a detector that detects a signal resulting from the analyte from the sensor device; and a processor that determines the concentration of the analyte in the liquid sample based on the signal.
Further, the present invention also provides a method for measuring an analyte in a liquid sample. The method includes the steps of: introducing the liquid sample into the sensor device; detecting a signal resulting from the analyte from the sensor device after bubbles produced in the liquid sample have reached the liquid sample level; and determining the concentration of the analyte in the liquid sample based on the signal.

### Effects of the Invention

The present invention can provide a sensor device that enables quick and uniform dissolution of reagents, has a simple structure, and is capable of size reduction.

### Brief Description of Drawings

FIG. 1 is an exploded perspective view of a sensor device according to Embodiment 1 of the present invention;
FIG. 2 is an exploded perspective view of a sensor device according to Embodiment 2 of the present invention;
FIG. 3 is an exploded perspective view of a sensor device according to Embodiment 4 of the present invention;
FIG. 4 is an exploded perspective view of a sensor device according to Embodiment 5 of the present invention;
FIG. 5 is a perspective view of a sensor device according to Embodiment 6 of the present invention;
FIG. 6 is a perspective view of a sensor device according to Embodiment 7 of the present invention;
FIG. 7 is a cross-sectional view taken along line A-A of FIG. 6;
FIG. 8 is an exploded perspective view of the sensor device according to Embodiment 7 of the present invention as illustrated in FIG. 6;
FIG. 9 is a perspective view of a modified example of the sensor device according to Embodiment 7 of the present invention;
FIG. 10 is a cross-sectional view taken along line B-B of FIG. 9;
FIG. 11 is an exploded perspective view of another modified example of the sensor device according to Embodiment 7 of the present invention; and
FIG. 12 is an exploded perspective view of still another modified example of the sensor device according to Embodiment 7 of the present invention.

### Best Mode for Carrying Out the Invention

The present invention relates to a sensor device that includes a holding member having a liquid sample holding section for holding a liquid sample containing an analyte. A reagent that reacts or interacts with the analyte in the liquid sample and an effervescent agent that effervesces upon contact with the liquid sample are held at a position of the holding member where they are to come into contact with the liquid sample introduced into the liquid sample holding section.
When a liquid sample is introduced to the liquid sample holding section of the sensor device of the present invention, the effervescent agent effervesces by contacting with the liquid sample. Hence, the dissolution of the reagent by the liquid sample can be readily promoted without supplying gas thereinto from outside the device. Also, due to the promotion, each time a measurement is made, the reagent can be dissolved uniformly, so that variations in measured values can be suppressed. In this way, the sensor device of the present invention enables simple, prompt, and highly accurate quantification.

Thus, the effervescent agent of the present invention produces bubbles, thereby physically mixing a liquid sample and the reagent as evenly as possible, without affecting reaction on quantification, such as enzyme reaction or immune reaction. It has a stirring function of evenly dissolving the reagent.
Also, unlike Patent Documents 3 and 4, a driving mechanism such as a motor or piezoelectric actuator for rotating a stirrer or vibrating a sensor is not necessary, so that the sensor device does not become complicated and large in size.

The holding member holds the reagent on at least a part of the surface thereof, and when a liquid sample is supplied thereto, the holding member can hold a mixture of the liquid sample and the reagent (reaction solution).
The holding member may be made of resin such as polyethylene terephthalate, polyacrylate, polymethyl methacrylate, or polypropylene.

When quantification of an analyte is performed electrochemically in the present invention, at least a part of the surface of the resin holding member is preferably coated with metal, a conductive polymer, inorganic matter, ink, or the like to form electrodes. In this case, current flowing between the electrodes or voltage can be linked with the analyte concentration for evaluation.

Also, when quantification of an analyte is performed spectroscopically in the present invention, at least a part of the resin is preferably transparent. In this case, information on absorption, transmission, scattering, reflection, diffraction, refraction, or the like of incident light passed through the transparent part by solution can be linked with the analyte concentration for evaluation.

Preferably, the effervescent agent used in the present invention produces bubbles mainly by producing carbon dioxide. Since carbon dioxide gas does not significantly interfere with common enzyme, immune, and other reactions, it hardly affects reaction occurring on quantification.
The effervescent agent is preferably a hydrogen carbonate or a carbonate. As shown in the following formula
(1) or formula (2), such a salt easily produces carbon dioxide when reacting with an acid.

MHCO₃ + HX → MX + CO₂ + H₂O (1)

M₂CO₃ + 2HX → 2MX + CO₂ + H₂O (2)

In the formulae (1) and (2), M represents an alkali metal atom such as Na or K, HX a weak acid, and MX an alkali metal salt.

Preferably, the sensor device including the effervescent agent further includes an organic acid. The organic acid functions as HX (acid) in the formulae (1) and (2). Since these acids are not volatile, they can be present in a solid or liquid state. Such an acid may be positioned apart from the hydrogen carbonate or carbonate. If in a dry solid state, it may be mixed with the hydrogen carbonate or carbonate. Both effervescent agent and organic acid may be contained in a reaction solution that is formed when a supplied liquid sample is mixed with the reagent. Also, the reagent or liquid sample may function as HX.

The organic acid is preferably at least one selected from the group consisting of maleic acid, maleic anhydride, succinic acid, succinic anhydride, phthalic acid, phthalic anhydride, citric acid, fumaric acid, and tartaric acid.
These organic acids and acids produced by hydrolysis of these acid anhydrides have a pKa (acid dissociation constant) range of 1.9 to 4.3, thereby facilitating and accelerating the production of bubbles by the acid-base reaction with the effervescent agent.

In the sensor device of the present invention, the reagent, the effervescent agent, and the organic acid can be present, if in a dry solid state, in the following forms of (1) to (5).
(1) a reagent layer, an effervescent agent layer, and an organic acid layer
(2) a mixture layer of reagent and organic acid, and an effervescent agent layer
(3) a mixture layer of reagent and effervescent agent, and an organic acid layer
(4) a mixture layer of effervescent agent and organic acid, and a reagent layer
(5) a mixture layer of reagent, effervescent agent, and organic acid

The reagent layer, the effervescent agent layer, the organic acid layer, and the mixture layer containing two or more of them as shown in (1) to (5) may be present at such positions that when a liquid sample is supplied, the reagent, the effervescent agent, and the organic acid can come into contact with the liquid sample on the holding member. For example, the respective layers of (1) to (5) may be laminated.

When the reagent layer and the effervescent agent layer are provided separately, it is preferable to arrange the effervescent agent layer upstream of the reagent layer. That is, it is preferable to arrange the effervescent agent layer and the reagent layer in such a positional relation that a liquid sample comes into contact with the effervescent agent layer before coming into contact with the reagent layer. In this case, the reagent comes into contact with the liquid sample containing a large amount of floating bubbles, thereby resulting in an improvement in the efficiency of dissolution of the reagent due to the effervescent action.
The equivalent ratio of the organic acid to the effervescent agent is preferably at least 1 or more. In this case, theoretically all the effervescent agent reacts, so that the utilization efficiency increases.

The effervescent agent layer can be obtained, for example, by shaping effervescent agent particles into film or pellet form. Specifically, it can be prepared by mixing effervescent agent particles with a suitable polymer powder and forming the mixture under pressure, or by dissolving or dispersing effervescent agent particles in a water-soluble polymer solution, dropping the resulting solution on the holding member, and drying it.

The reagent layer can be prepared, for example, by dropping a reagent-containing solution on the holding member and drying it. When the reagent-containing solution tends to flow and spread on the holding member, for example, the holding member may be provided with a recess and the reagent solution may be dropped in the recess and dried. The drying may be freeze-drying or heat-drying at an air temperature of 30 to 50 °C. The reagent solution is preferably a solution of a water-soluble polymer, since the solution has improved viscosity and the reagent layer can be easily formed.
The organic acid layer can be formed by the same formation methods as those of the reagent layer. Also, a mixture layer of a reagent and an organic acid may be formed by adding the organic acid to a solution of the reagent and drying it.

The liquid sample to be measured by the sensor device of the present invention is preferably a liquid derived from a living body, such as blood, blood plasma, blood serum, interstitial fluid, urine, or saliva. The quantification of a specific component contained in these liquids can be performed readily by using the sensor device of the present invention.
The reagent that reacts or interacts with an analyte in a liquid sample is preferably an enzyme, an antibody, or a combination thereof. Since these reagents have good substrate (reaction) selectivity and affinity (binding) selectivity with respect to a specific compound, they are advantageous for quantification with the sensor device of the present invention.

When an enzyme and an antibody are combined, for example, an enzyme layer and an antibody layer may be formed and these two layers may be laminated. Alternatively, a mixture layer of an enzyme and an antibody may be formed.
The enzyme can be selected as appropriate depending on the substrate contained in a liquid sample. Usable examples include fructose dehydrogenase, glucose oxidase (GOD), PQQ-dependent glucose dehydrogenase, NAD-dependent glucose dehydrogenase, alcohol oxidase, lactic acid oxidase, cholesterol oxidase, xanthine oxidase, and amino acid oxidase.

The method of measuring an analyte in a liquid sample with the sensor device of the present invention can be, for example, the quantification of an analyte by oxidizing an electron mediator on an electrode and measuring the resulting oxidation current. The electron mediator can be potassium ferricyanide, p-benzoquinone, phenazine methosulfate, methylene blue, a ferrocene derivative, a Ru or Os complex, or the like. Also, even when the electron mediator is oxygen, response current can be obtained as well. Also, they may be used singly or in combination of two or more of them.
Further, in the case of using an electron mediator whose color changes due to oxidation/reduction, such as potassium ferricyanide, phenazine methosulfate, or methylene blue, spectroscopic measurement can be performed, for example, by measuring absorbance change.

With respect to the electrode, at least a working electrode and a counter electrode are used. The electrode may be made of any material if the material is a conductive substance that is not oxidized itself. The electrode is preferably produced, for example, by screen printing, sputtering, or evaporation.

Further, an analyte (antigen) in a liquid sample may be measured by utilizing an antibody as the reagent for the sensor device of the present invention and utilizing immune reaction. The antigen is not particularly limited and may be any substance that can be usually measured by utilizing antigen-antibody reaction. Examples include proteins, nucleic acids, lipids, bacteria, viruses, and haptens. Among them, proteins are preferred since they are substances mainly measured in clinical tests using antigen-antibody reaction.

Examples of proteins include hormones, such as LH (luteinizing hormone), FSH (follicle-stimulating hormone), and hCG (human-chorionic gonadotropin), various immonoglobulin classes, subclasses thereof, complement components, markers for various infectious diseases, C-reactive protein (hereinafter referred to as CRP), albumins, rheumatoid factors, and blood group antigens. Among them, human albumin or human CRP is preferred.

The antibody is not particularly limited and may be any antibody capable of specifically binding to its antigen. For example, it may be an antibody of any class, such as IgG, IgM, IgE, IgA, or IgD. Among them, IgG antibody is preferred since it has less non-specific reactivity, is relatively often commercially available, and is easily obtainable. Further, the species of an animal from which an antibody is derived is not particularly limited, but an antibody derived from a rabbit, goat or mouse is preferred since it is relatively easily obtainable and generally used.

Also, the above-mentioned reagent can further include other optional component known in the art depending on the application or the like, as long as the effects of the present invention can be obtained. For example, in the case of homogenous immunoreaction assays such as nephelometry, turbidimetry, and slide agglutination, the reagent of the present invention may further include polyethylene glycol.

In immunoreaction assay using the sensor device of the present invention, the antigen-antibody complex produced by immunoreaction is preferably an agglutination complex. Also, the agglutination complex is preferably detected by measuring an optical characteristic derived from the agglutination complex. The optical characteristic is preferably scattered light intensity or transmitted light intensity.

In the present invention, at least a part of the inner face of the liquid sample holding section is preferably hydrophilic. For example, by applying a hydrophilic treatment to a part of the inner wall of the liquid sample holding section to come into contact with a liquid sample introduced into the liquid sample holding section, the part can be made hydrophilic. In this case, bubbles produced by the effervescent agent are prevented from remaining on the surface of the liquid sample holding section and the produced bubbles can be removed from the liquid sample. As a result, it is possible to obtain the effect of dissolving the reagents more evenly and sufficiently.

In the liquid sample holding section, particularly the measurement section for measuring an analyte in a liquid sample is preferably hydrophilic. The measurement section can be, for example, in the case of a sensor device for electrochemical measurement, an electrode section having conductive area, or in the case of a sensor device for optical measurement, a light inlet section through which light enters from a light source and/or a light outlet section through which the light having entered the liquid sample holding section exits. In this case, bubbles produced by the effervescent agent are prevented from remaining on the measurement section and the bubbles are prevented from interfering with the measurement. As a result, it is possible to perform more accurate and highly reproducible measurement.
Embodiments of the present invention are hereinafter described in detail.

### 〈〈Embodiment 1〉〉

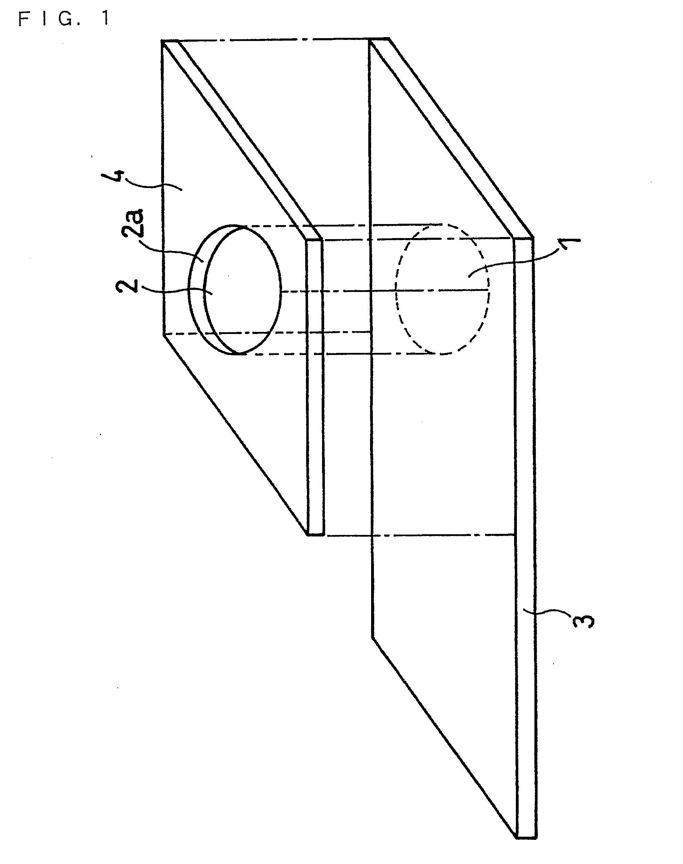

A glucose sensor is described as one embodiment of the sensor device. FIG. 1 is an exploded perspective view of a glucose sensor according to Embodiment 1 of the sensor device of the present invention.
The glucose sensor of this embodiment includes a holding member that is obtained by bonding an insulating second base plate 4 with a hole 2 onto an insulating first base plate 3 as illustrated in FIG. 1. The first base plate 3 is colorless and transparent and made of, for example, polystyrene or polymethyl methacrylate, and the second base plate 4 is made of, for example, polyethylene terephthalate. This holding member has a recess serving as a liquid sample holding section 1, the recess being formed on the first base plate 3 in the hole 2 of the second base plate 4.
The glucose sensor is prepared, for example, by dropping an aqueous solution containing GOD as an enzyme, 1-methoxy-5-methylphenazinium (hereinafter referred to as MMP) as an electron mediator, and succinic acid as an organic acid on the liquid sample holding section 1 of the holding member, drying it to form a reagent layer, and spraying sodium hydrogen carbonate powder as an effervescent agent onto the reagent layer.

Using this glucose sensor, the glucose concentration in a glucose aqueous solution can be measured, for example, as follows. A glucose aqueous solution with a glucose concentration of approximately 360 mg/dl is supplied to the liquid sample holding section 1. Then, bubbles are produced by the effervescent agent. After 10 seconds, light of 620 nm, which is the wavelength of absorption peak of oxidized form MMP, is vertically irradiated on the base plate 3, and the absorbance is measured with an absorptiometer. Further, after a predetermined period of time, the absorbance is again measured. At this time, in the reaction solution, glucose is oxidized and MMP is reduced, so the absorbance decreases with time. The amount of absorbance decrease is dependent on the glucose concentration in the liquid sample.

The glucose sensor according to this embodiment has the following effects in comparison with conventional glucose sensors including neither sodium hydrogen carbonate, which is an effervescent agent, nor succinic acid, which is an organic acid.
First, in the glucose sensor of this embodiment, since the reagents such as GOD and MMP dissolve quickly and fully, a large amount of glucose reacts, so that the amount of absorbance decrease is greater than that in conventional glucose sensors.

Also, in the glucose sensor of this embodiment, since the reagents dissolve quickly and evenly, variations in absorbance decrease are suppressed relative to those in conventional glucose sensors.
As described above, in the glucose sensor including the effervescent agent, the dissolution of the reagents is promoted and the dissolution state is well reproduced. Therefore, the glucose sensor including the effervescent agent is capable of prompt and highly accurate measurement.
In the above description, light with a wavelength of 620 nm was irradiated in order to measure the amount of the electron mediator. However, since this wavelength changes depending on the electron mediator, light with an optimum wavelength may be irradiated, as needed.

In order to further facilitate the supply of a liquid sample to the reagent layer, a hydrophilic layer may be formed by applying a solution of lecithin or Triton X-100 in toluene or other organic solvent onto an inner wall 2a in the hole 2 of the second base plate 4, which faces the liquid sample holding section 1, and drying it. In this case, the amount of liquid sample supplied to the reagent layer can be kept constant and more accurate quantification becomes possible.

As in this embodiment, a sensor in which the surface of the liquid sample holding section is subjected to a hydrophilic treatment with lecithin or the like is superior to a sensor without such treatment in terms of the reproducibility and accuracy of measurement results. Particularly, the bubbles produced by the effervescent agent are prevented from remaining on the surface of the liquid sample holding section and the produced gas can be removed from the liquid sample. It is thus possible to obtain the effect of dissolving the reagents more evenly and sufficiently.

Also, if a hydrophilic treatment is applied to a measuring part of the liquid sample holding section, for example, an electrode section in the case of electrochemical measurement or light inlet/outlet sections in the case of optical measurement, the bubbles produced by the effervescent agent are prevented from remaining on the measuring part. It is thus possible to perform more accurate and highly reproducible measurement.
The compound used in the hydrophilic treatment is not limited to lecithin or Triton X-100 and may be any material that can make an interface hydrophilic.

### 〈〈Embodiment 2〉〉

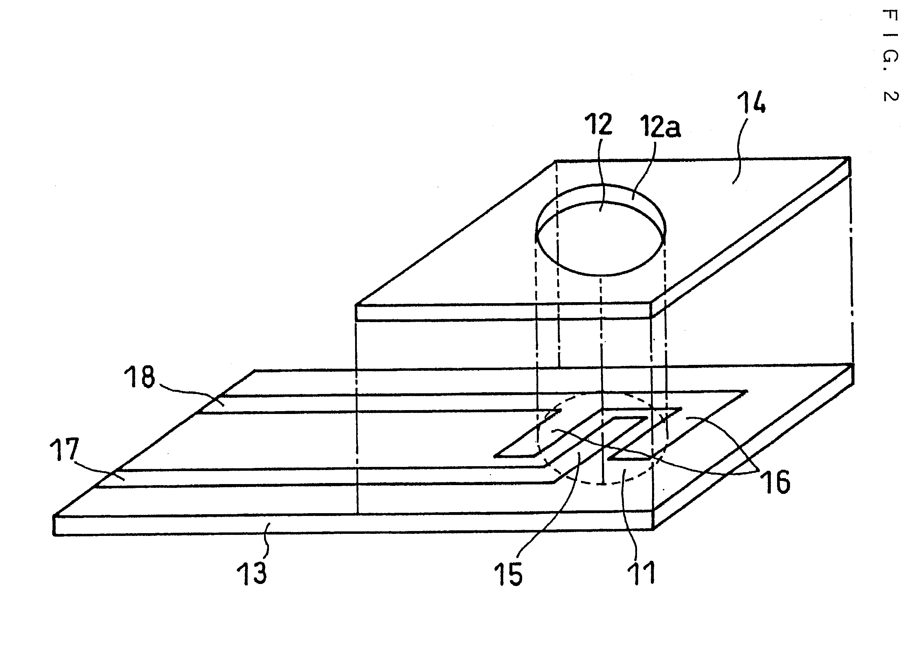

In this embodiment, a description is made of a glucose sensor that quantifies glucose by electrochemical measurement. FIG. 2 is an exploded perspective view of a glucose sensor according to Embodiment 2 of the sensor device of the present invention.
The glucose sensor according to this embodiment includes a holding member obtained by bonding a first base plate 13 and a second base plate 14. The insulating first base plate 13 made of, for example, polyethylene terephthalate has thereon an electrode section composed of a working electrode 15 and a counter electrode 16, and leads 17 and 18. The electrode section and the leads 17 and 18 are prepared, for example, by placing a resin mask of electrode pattern on the first base plate 13 and sputtering gold. The working electrode 15 and the counter electrode 16 are electrically connected to measuring terminals outside the glucose sensor by the leads 17 and 18, respectively.

The holding member is obtained by bonding the insulating second base plate 14 onto the first base plate 13 as illustrated in FIG. 2. The insulating second base plate 14 is made of, for example, polyethylene terephthalate and has a hole 12, which forms a liquid sample holding section 11 for holding reagents. The electrode section is exposed in the liquid sample holding section 11.
The glucose sensor is prepared, for example, by dropping an aqueous solution containing GOD as an eyzyme, potassium ferricyanide as an electron mediator, and succinic acid as an organic acid on the liquid sample holding section 11 of the holding member, drying it to form a reagent layer, and spraying sodium hydrogen carbonate powder as an effervescent agent onto the reagent layer.

Using this glucose sensor, for example, the glucose concentration in a glucose aqueous solution can be measured, as follows. A glucose aqueous solution with a glucose concentration of approximately 360 mg/dl is supplied to the liquid sample holding section 11. Then, bubbles are produced by the effervescent agent. After 10 seconds, a voltage of 500 mV is potentiostatically applied between the working electrode 15 and the counter electrode 16, with the working electrode being positive. Then, 5 seconds after the application of the voltage, the value of current flowing between the working electrode and the counter electrode is measured. In the reaction solution, first, glucose reacts with GOD and ferricyanide ions are reduced to ferrocyanide ions. Due to the application of the voltage, the ferrocyanide ions are oxidized on the working electrode, whereby a response current is obtained. The response current increases as the glucose concentration in the liquid sample increases.

The glucose sensor according to this embodiment can also produce the same effects as that of Embodiment 1 in comparison with conventional glucose sensors including neither sodium hydrogen carbonate, which is an effervescent agent, nor succinic acid, which is an organic acid.
As described above, in the glucose sensor including the effervescent agent, the dissolution of the reagents is promoted and the dissolution state is well reproduced. Therefore, the glucose sensor including the effervescent agent is capable of prompt and highly accurate measurement.
In the above description, an example of the glucose sensor for electrochemical measurement has been shown, but the shape, number, arrangement, or the like of the electrodes and leads disposed in the sensor are not limited to this. Also, the voltage applied to the electrode system to obtain a response current was 500 mV, but the voltage is not limited to this value. The voltage may be any voltage at which there is a change in electrical signal and the electron mediator is oxidized.

In this embodiment, also, in order to further facilitate the supply of a liquid sample to the reagent layer, a hydrophilic layer may be formed by applying a solution of lecithin or Triton X-100 in toluene or other organic solvent onto an inner wall 12a in the hole 12 of the second base plate 14, which faces the liquid sample holding section 11, and drying it. In this case, the amount of liquid sample supplied to the reagent layer can be kept constant and more accurate quantification becomes possible.

As in this embodiment, a sensor in which the surface of the liquid sample holding section is subjected to a hydrophilic treatment with lecithin or the like is superior to a sensor without such treatment in terms of the reproducibility and accuracy of measurement results. Particularly, the bubbles produced by the effervescent agent are prevented from remaining on the surface of the liquid sample holding section and the produced gas can be removed from the liquid sample. It is thus possible to obtain the effect of dissolving the reagents more evenly and sufficiently.

Also, if a hydrophilic treatment is applied to a measuring part of the liquid sample holding section, for example, an electrode section in the case of electrochemical measurement or light inlet/outlet sections in the case of optical measurement, the bubbles produced by the effervescent agent are prevented from remaining on the measuring part. It is thus possible to perform more accurate and highly reproducible measurement.
The compound used in the hydrophilic treatment is not limited to lecithin or Triton X-100 and may be any material that can make an interface hydrophilic.

### 〈〈Embodiment 3〉〉

In this embodiment, a sensor device having the same structure as that of the sensor device of Embodiment 1 is used to describe an albumin sensor for measuring human albumin by immunonephelometry.
First, a reagent comprising an antibody that is usable in measurement by nephelometry can be prepared as follows. This reagent can also be used in measurements by slide agglutination and turbidimetry in addition to nephelometry.

An antibody solution used as the reagent can be prepared, for example, by the following method.
An antiserum collected from rabbits immunized with human albumin (available from Wako Pure Chemical Industries, Ltd.) is purified by protein A column chromatography to obtain rabbit anti-human albumin polyclonal antibody.
Purification is performed, for example, as follows. A gel is filled into a column, and an equilibration buffer having a volume 5 times that of the gel is passed through the column to equilibrate the column. Thereafter, an antiserum containing antibodies at 10 to 20 % of the overall binding capacity of the column is double diluted with the equilibration buffer, and then passed through the column to allow the antibodies in the serum to bind to protein A. At this time, the gel used in the chromatography column is protein A immobilized gel available from Amersham-Pharmacia. Also, the equilibration buffer may be a pH 8.9 buffer containing 1.5 M of glycine and 3.0 M of NaCl.

Subsequently, the equilibration buffer is passed therethrough until no serum component not adsorbed onto the protein A comes out of the column, to wash the column. Thereafter, an elution buffer is passed through the column to elute the anitibodies bound to the protein A. The elution buffer is a pH 4.0 buffer containing, for example, 0.1 M of citric acid.
The eluted antibody fraction is placed into a dialysis tube with a molecular weight cut-off of 10000, and dialysis was performed several times using an about 100-fold volume of a pH 7.4 buffer containing 0.05 M of 3-(N-morpholino)propanesulfonic acid (available from Dojin, hereinafter referred to as MOPS), 0.15 M of NaCl, and 0.04% by weight of NaN₃, to replace the buffer components.

Subsequently, the antibody concentration is estimated by 280 nm absorptiometric analysis, and the antibody concentration is adjusted to 3.0 mg/ml by using the same buffer as used in the dialysis. In this way, an antibody solution can be prepared.
It should be noted that the concentration of the antibody solution is not particularly limited to this. The antibody solution is stored in an environment at 4°C. The antibody solution may be stored at room temperature, but is preferably stored at low temperature in order to prevent denaturation of the antibody.

An albumin sensor can be prepared by using the same sensor device as that of Embodiment 1, dropping a solution containing the thus obtained antibody solution, polyethylene glycol 6000 (hereinafter referred to as PEG), and succinic acid on the liquid sample holding section 1, drying it to form a reagent layer, and spraying sodium hydrogen carbonate on the reagent layer as the effervescent agent.

Using this albumin sensor and using a human albumin solution as a liquid sample, measurement can be carried out, for example, by the following method. Using a buffer of pH 7.4 containing 0.05 M of MOPS and 0.04% by weight of NaN₃, the concentration of human albumin (available from Wako Pure Chemical Industries, Ltd.) solution used as an antigen is adjusted to 0, 5, 10, 20, 30, 50, 70, 100, 200, 300, or 500 mg/dl. By supplying a human albumin solution to the albumin sensor, bubbles are produced by the effervescent agent. Using a spectrophotometer, light with a wavelength of 670 nm is vertically irradiated on the base plate 3 and measurement is made for 60 seconds.

The average value of the respective measured values (scattered light intensities) obtained during 30 to 45 seconds is calculated, and this is used as the measured value for the human albumin solution at each concentration.
In the reaction solution, the binding reaction between the antigen and the antibody produces agglutination, and the scattered light intensity increases with time. The measured value for the human albumin solution at each concentration depends on the human albumin concentration in the liquid sample.

The albumin sensor according to this embodiment has the following effects in comparison with conventional albumin sensors including neither sodium hydrogen carbonate, which is an effervescent agent, nor succinic acid, which is an organic acid.
First, measured values obtained from the alubumin sensor of this embodiment are greater than measured values obtained from conventional sensors, since the reagents comprising the antibody and PEG dissolve sufficiently due to the effervescent agent, thereby facilitating the binding of the antibody to the antigen.

Also, in the albumin sensor of this embodiment, since the reagents dissolve quickly and evenly, variations in measured values are suppressed relative to those in conventional alubumin sensors.
As described above, even when the antibody is used as a reagent in the alubumin sensor including the effervescent agent, the dissolution of the reagent is promoted and the dissolution state is well reproduced. Therefore, the alubumin sensor including the effervescent agent is capable of prompt and highly accurate measurement.
In the above description, light with a wavelength of 670 nm was used in the measurement utilizing immune reaction, but there is no limitation thereto, and light with any wavelength may be used if scattering of light by agglutination resulting from antigen-antibody reaction can be observed.

### 〈〈Embodiment 4〉〉

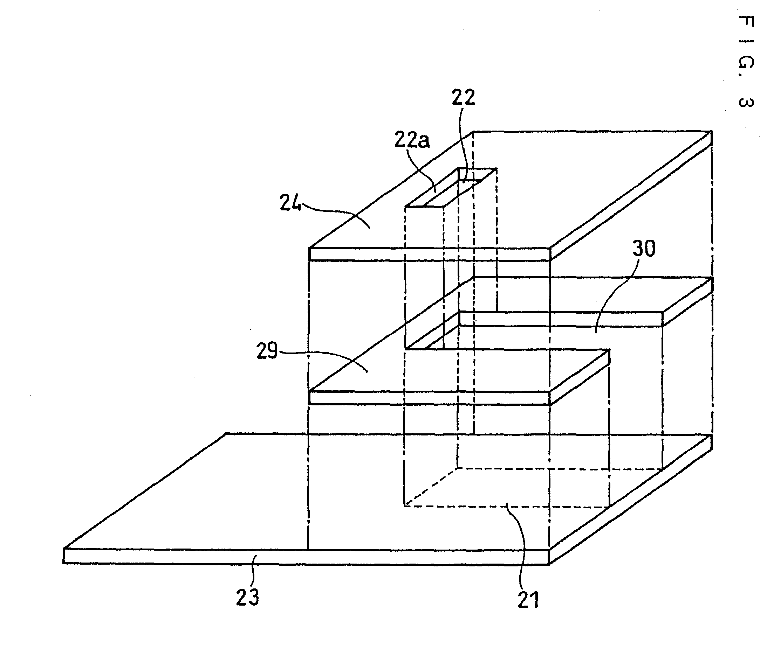

In Embodiment 4 of the sensor device of the present invention, a holding member as illustrated in FIG. 3 may also be used. The holding member is prepared by bonding a third base plate 29, which has a slit 30 for forming a liquid sample holding section 21, to a first base plate 23, and bonding a second base plate 24, which has a hole 22 communicating with the liquid sample holding section 21 and is colorless and transparent and made of, for example, polymethyl methacrylate, to the base plate 29. The hole 22 can also serve as a part of the liquid sample holding section 21. FIG. 3 is an exploded perspective view of a sensor device according to Embodiment 4 of the present invention.
When this sensor device is provided with the same reagent layer and effervescent agent as those in Embodiments 1 and 3 and is used to perform measurements by the same methods as those in Embodiments 1 and 3, essentially the same effects as those of Embodiments 1 and 3 can be obtained as well.

In this embodiment, also, in order to further facilitate the supply of a liquid sample to the reagent layer, a hydrophilic layer may be formed by applying a solution of lecithin or Triton X-100 in toluene or other organic solvent onto an inner wall 22a in the hole 22 of the second base plate 24, which faces the liquid sample holding section 21, and drying it. In this case, the amount of liquid sample supplied to the reagent layer can be kept constant and more accurate quantification becomes possible.

As in this embodiment, a sensor in which the surface of the liquid sample holding section is subjected to a hydrophilic treatment with lecithin or the like is superior to a sensor without such treatment in terms of the reproducibility and accuracy of measurement results. Particularly, the bubbles produced by the effervescent agent are prevented from remaining on the surface of the liquid sample holding section and the produced gas can be removed from the liquid sample. It is thus possible to obtain the effect of dissolving the reagents more evenly and sufficiently.

Also, if a hydrophilic treatment is applied to a measuring part of the liquid sample holding section, for example, an electrode section in the case of electrochemical measurement or light inlet/outlet sections in the case of optical measurement, the bubbles produced by the effervescent agent are prevented from remaining on the measuring part. It is thus possible to perform more accurate and highly reproducible measurement.
The compound used in the hydrophilic treatment is not limited to lecithin or Triton X-100 and may be any material that can make an interface hydrophilic.

### 〈〈Embodiment 5〉〉

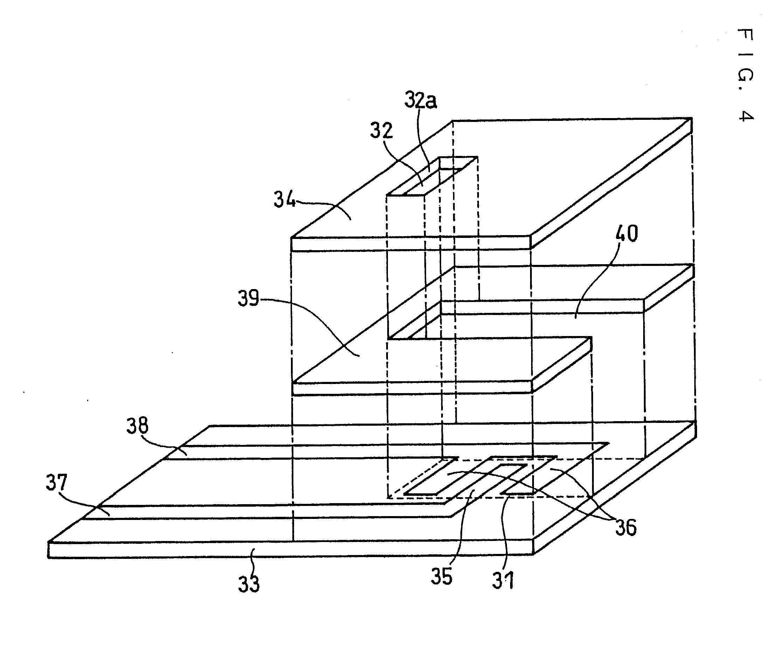

Also, when an electrochemical measurement is performed as in Embodiment 2, a holding member as illustrated in FIG. 4 may also be used as Embodiment 5 of the sensor device of the present invention. The holding member includes a third base plate 39, which has a slit 40 for forming a liquid sample holding section 31 where a working electrode 35 and a counter electrode 36 are exposed on a first base plate 33, and a second base plate 34, which has a hole 32 communicating with the liquid sample holding section 31. The hole 32 can also serve as a part of the holding section 31.
FIG. 4 is an exploded perspective view of a sensor device according to Embodiment 5 of the present invention.

In order to further facilitate the supply of a liquid sample to the reagent layer, a hydrophilic layer may be formed by applying a solution of lecithin or Triton X-100 in toluene or other organic solvent onto an inner wall 32a in the hole 32 of the second base plate 34, and drying it. In this case, the amount of liquid sample supplied to the reagent layer can be kept constant and more accurate quantification becomes possible.

As in this embodiment, a sensor in which the surface of the liquid sample holding section is subjected to a hydrophilic treatment with lecithin or the like is superior to a sensor without such treatment in terms of the reproducibility and accuracy of measurement results. Particularly, the bubbles produced by the effervescent agent are prevented from remaining on the surface of the liquid sample holding section and the produced gas can be removed from the liquid sample. It is thus possible to obtain the effect of dissolving the reagents more evenly and sufficiently.

Also, if a hydrophilic treatment is applied to a measuring part of the liquid sample holding section, for example, an electrode section in the case of electrochemical measurement or light inlet/outlet sections in the case of optical measurement, the bubbles produced by the effervescent agent are prevented from remaining on the measuring part. It is thus possible to perform more accurate and highly reproducible measurement.
The compound used in the hydrophilic treatment is not limited to lecithin or Triton X-100 and may be any material that can make an interface hydrophilic.

### 〈〈Embodiment 6〉〉

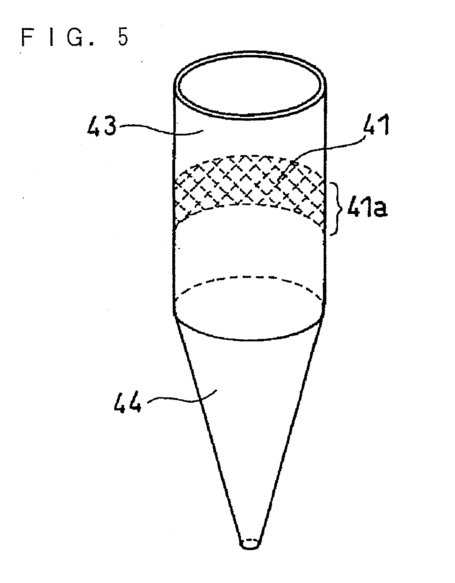

Further, a sensor device comprising a hollow holding member as illustrated in FIG. 5 may also be used as Embodiment 6 of the present invention. This sensor device includes a holding member that is prepared by combining a cylindrical first hollow structure 43, which has a liquid sample holding section 41 on the inner wall, and a conical second hollow structure 44, which is joined to the hollow structure 43 such that the bottom of the hollow structure 43 is corresponded to the bottom of the hollow structure 44. The material of this sensor device can be methyl methacrylate. Also, the shape of the sensor device may be in the shape of a polygonal tube or pyramid. FIG. 5 is a perspective view of the sensor device according to Embodiment 6 of the present invention.

For example, the sensor device of this embodiment can be prepared by disposing the reagent layer and effervescent agent of Embodiment 1 on at least a part of the inner wall of the liquid sample holding section 41 in the hollow. For example, the reagent layer can be formed on a section 41a of the liquid sample holding section 41 by placing the cylinder of the holding member such that its symmetry axis is perpendicular to the gravity direction, dropping the reagent solution by using a syringe with a long needle or the like on the liquid sample holding section 41, and drying it.

Also, absorbance can be measured in the same manner as in Embodiment 1 by sucking the same glucose aqueous solution as that of Embodiment 1 up to at least the position of the section 41a where the reagent layer is formed, until a certain amount of the liquid sample is contained therein, through the opening of the second hollow structure 44, by using a micropump (not shown) connected to the first hollow structure 43.

Further, scattered light intensity can also be measured in the same manner as in Embodiment 3 by disposing the reagent layer and effervescent agent of Embodiment 3 on at least the section 41a of the wall in the liquid sample holding section 41 of the holding member of FIG. 5 and sucking the human albumin solution of Embodiment 3 up to at least the section 41a where the reagent layer is disposed, until a certain amount of the liquid sample is contained therein, by using a micropump. In performing such an optical measurement, light may be passed through the liquid sample holding section 41. However, the hollow structure 43 may preferably include optical windows with a controlled optical path length, through which light is passed and detected.

In order to further facilitate the supply of a liquid sample to the reagent layer, a hydrophilic layer may be formed by applying a solution of lecithin or Triton X-100 in toluene or other organic solvent onto any area of the inner walls of the first hollow structure 43 and the second hollow structure 44 (particularly preferably, the area from near the hole at the tip end of the second hollow structure 44 to the section 41a where the reagent layer is disposed), and drying it. In this case, the flow of the liquid sample supplied to the reagent layer can be stabilized and more accurate quantification becomes possible.

As in this embodiment, when a liquid sample is sucked up into the liquid sample holding section, a sensor in which the surface of the liquid sample holding section is subjected to a hydrophilic treatment with lecithin or the like is superior to a sensor without such treatment in terms of the reproducibility and accuracy of measurement results. Particularly, the bubbles produced by the effervescent agent are prevented from remaining on the surface of the liquid sample holding section and the produced gas can be removed from the liquid sample. It is thus possible to obtain the effect of dissolving the reagents more evenly and sufficiently.

Also, if a hydrophilic treatment is applied to a measuring part of the liquid sample holding section, for example, an electrode section in the case of electrochemical measurement or light inlet/outlet sections in the case of optical measurement, the bubbles produced by the effervescent agent are prevented from remaining on the measuring part. It is thus possible to perform more accurate and highly reproducible measurement.
The compound used in the hydrophilic treatment is not limited to lecithin or Triton X-100 and may be any material that can make an interface hydrophilic.

### 〈〈Embodiment 7〉〉

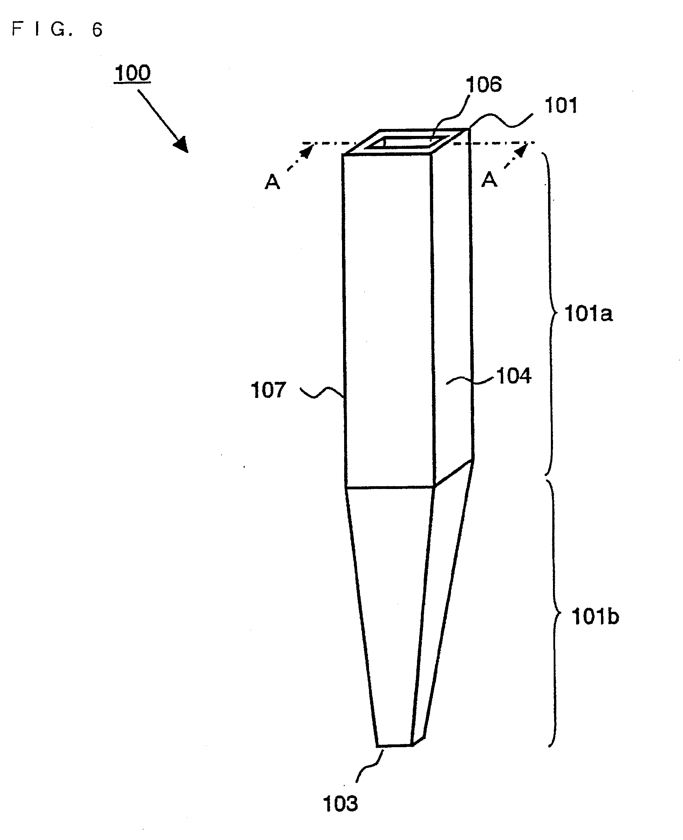

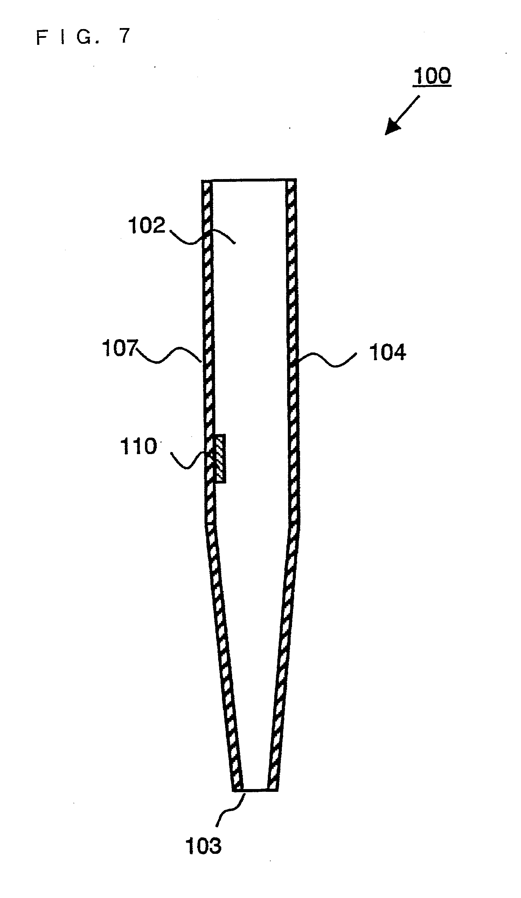

Referring to FIG. 6, the structure of a sensor device according to Embodiment 7 is described. FIG. 6 is a perspective view of the sensor device according to this embodiment, and FIG. 7 is a cross-sectional view taken along line A-A of FIG. 6.
A sensor device 100 of this embodiment has a hollow holding member 101 made of transparent polystyrene (hereinafter also referred to as a "body"). The body 101 is open at both ends thereof, and there is a space inside the body 101. The space serves as a liquid sample holding section 102.

Also, one open end of the space serving as the liquid sample holding section 102 serves as a sample supply inlet 103, while the other open end serves as a sucking port 106.
More specifically, the body 101 has a hollow quadrangular prismatic section 101a and a hollow quadrangular pyramidal section 101b. One end of the hollow quadrangular prismatic section 101a is provided with the sucking port 106, while the other end of the hollow quadrangular prismatic section 101a is integrated with the hollow quadrangular pyramidal section 101b. The end of the hollow quadrangular pyramidal section 101b opposite to the hollow quadrangular prismatic section 101a is provided with the sample supply inlet 103.

Of the four faces constituting the exterior of the hollow quadrangular prismatic section 101a, a face 107 serves as a light inlet section (hereinafter also referred to as a "light inlet section 107"), and a face 104 serves as a light outlet section (hereinafter also referred to as a "light outlet section 104"). The face 107 serving as the light inlet section and the face 104 serving as the light outlet section constitute an optical measurement section in this embodiment. Also, of the inner walls surrounding the liquid sample holding section 102, the inner wall of the face 107 of the hollow quadrangular prismatic section 101a is provided with a reagent holding section 110.
When the sensor device 100 according to this embodiment is used, a part of the sensor device 100 is immersed in, for example, urine taken into a container, and the urine is then sucked into the liquid sample holding section 102 through the sucking inlet 104 by a measuring apparatus, as described later.

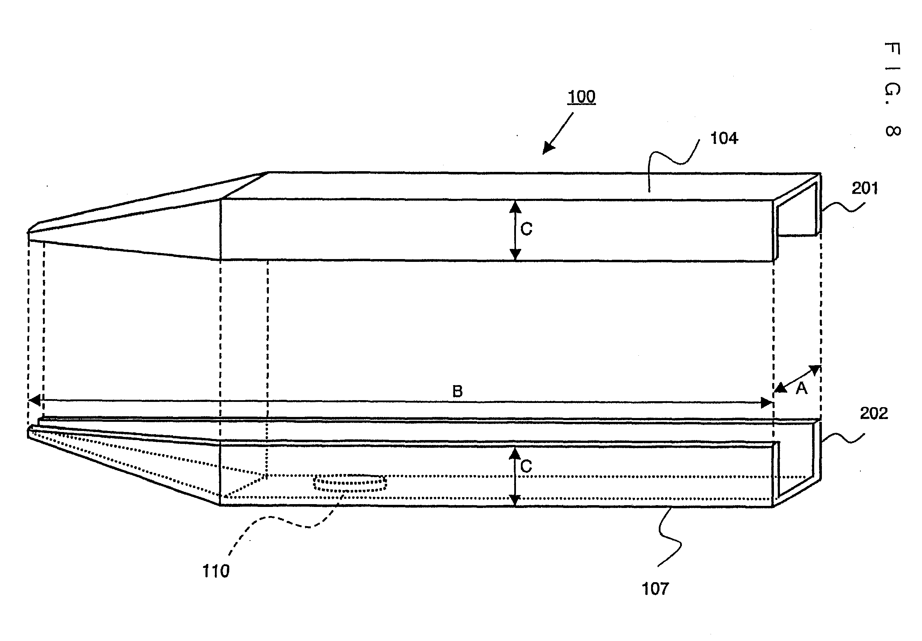

Referring now to FIG. 8, a method for producing the sensor device of this embodiment is described. FIG. 8 is an exploded perspective view of the sensor device according to this embodiment.
The sensor device 100 is composed of a first member 201 and a second member 202, each of which is made of transparent polystyrene and has a recess. The first member 201 and the second member 202 are combined with each other to form the body 101 having the hollow quadrangular prismatic section 101a and the hollow quadrangular pyramidal section 101b.
The first member 201 and the second member 202 can be molded by using a mold. Any known resin molding technique may be used for this molding. The dimensions of the first member 201 and the second member 202 can be freely adjusted. For example, the width A is 10 mm, the length B 84 mm, and the height C 6 mm.

Subsequently, the reagent holding section (reagent layer containing an effervescent agent) 110 is formed on the bottom of the recess of the second member 202, i.e., on the inner wall of the face 107. For example, a given amount of an aqueous solution of an antibody against human albumin, which is a reagent for optical measurement, is dropped on the bottom of the recess of the second member 202 by using a microsyringe or the like. The solution is allowed to stand in an environment at room temperature to about 30°C to evaporate the water content. In this way, the reagent can be carried thereon in a dry state. For example, an aqueous solution of the above-mentioned antibody with a concentration of 8 mg/dL may be used, and 0.7 mL of the solution may be dropped on an area of 5 cm². Thereafter, the solution is dried to form a reagent layer, and sodium hydrogen carbonate powder is sprayed on the reagent layer as the forming agent. In this way, the reagent holding section 110 can be obtained.

The concentration and amount of the aqueous solution containing the reagent applied, the amount of the effervescent agent, or the like can be appropriately selected depending on the characteristics of an intended device and the space restriction on the position of the reagent holding section 110 formed on the second member 202. Also, the position and area of the reagent holding section 110 formed on the second member 202 can be appropriately selected, as needed, in view of the solubility of the reagent relative to the liquid sample, the position of the optical measurement section, or the like.

The first member 201 and the second member 202 thus obtained are joined in a positional relation as shown by the broken line of FIG. 8, to fabricate the sensor device 100 as illustrated in FIGs. 6 and 7. The sensor device 100 is fabricated by applying an adhesive such as epoxy resin onto the joint between the first member 201 and the second member 202, bonding the first member 201 and the second member 202 together, and leaving and drying the adhesive.
It is also possible to laminate the first member 201 and the second member 202 without applying an adhesive and then thermally or ultrasonically weld the joint between the first member 201 and the second member 202 by using a commercially available welding device. In this way, the sensor device 100 as illustrated in FIGs. 6 and 7 can be obtained.

In order to further facilitate the supply of a liquid sample to the reagent layer, a hydrophilic layer may be formed by applying a solution of lecithin or Triton X-100 in toluene or other organic solvent onto the inner wall of the body 101 (i.e., wall of the liquid sample holding section 102) in any area thereof (particularly preferably, the area from near the sample supply inlet 103 to the reagent holding section 110), and drying it. In this case, the amount of liquid sample supplied to the reagent layer can be kept constant and more accurate quantification becomes possible.

As in this embodiment, when a liquid sample is sucked into a liquid sample holding section, a sensor in which the surface of the liquid sample holding section is subjected to a hydrophilic treatment with lecithin or the like is superior to a sensor without such treatment in terms of the reproducibility and accuracy of measurement results. Particularly, the bubbles produced by the effervescent agent are prevented from remaining on the surface of the liquid sample holding section and the produced gas can be removed from the liquid sample. It is thus possible to obtain the effect of dissolving the reagents more evenly and sufficiently.

Also, if a hydrophilic treatment is applied to a measuring part of the liquid sample holding section, for example, an electrode section in the case of electrochemical measurement or light inlet/outlet sections in the case of optical measurement, the bubbles produced by the effervescent agent are prevented from remaining on the measuring part. It is thus possible to perform more accurate and highly reproducible measurement.
The compound used in the hydrophilic treatment is not limited to lecithin or Triton X-100 and may be any material that can make an interface hydrophilic.

In the above description, an example of the embodiment of the present invention has been described, and the shape of the sensor device 100 is not limited to that as described in the above embodiment if it satisfies the requirements of the present invention and produces the effects of the present invention.

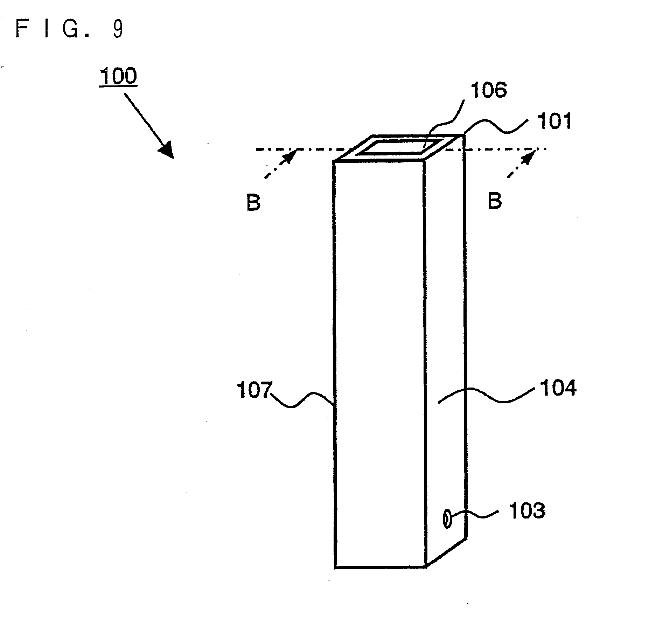

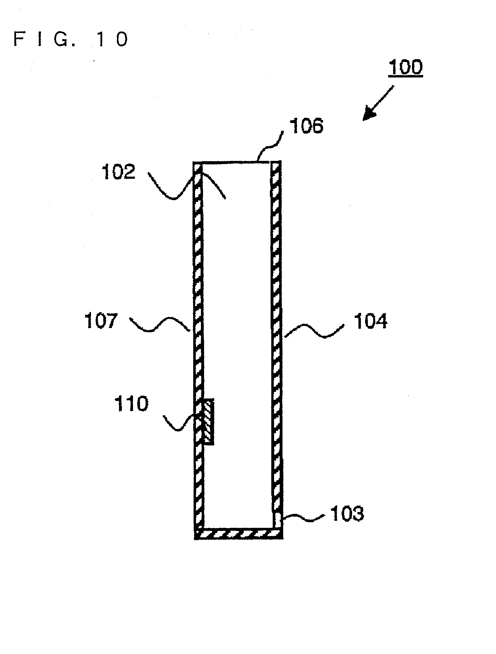

FIG. 9 is a perspective view of a modified example of the sensor device of the above embodiment, and FIG. 10 is a cross-sectional view taken along B-B of FIG. 9. The same constituent elements as those of FIGs. 6 and 7 are given the same reference characters and their description is omitted.

As illustrated in FIG. 9, the sensor device 100 of the modified example of the present invention includes the hollow rectangular body 101 having a space serving as the liquid sample holding section 102 and a bottom, and the face 104 of the body 101 is provided with the sample supply inlet 103. Also, the inner wall of the face 107 is provided with the reagent holding section 110.

In the description of the above embodiment, the reagent holding section 110 is formed by applying the aqueous solution containing the reagent for optical measurement and drying it; instead, the reagent holding section may be formed by impregnating a porous carrier made of glass fiber, filter paper, or the like with the reagent solution, drying or freeze-drying it to carry the reagent, dispersing the effervescent agent thereon, and affixing the carrier to the inner wall of the liquid sample holding section.

Also, the sensor device of this embodiment has the sucking port 106 for sucking a sample into the liquid sample holding section through the sample supply inlet. In making various measurements by using the sensor device of the present invention and a measuring apparatus, the sensor device is mounted such that the sucking port of the sensor device is connected to a sensor-device mounting section of the measuring apparatus. In this state, air inside the liquid sample holding section of the sensor device is sucked by a suction unit of the measuring apparatus through the sucking port of the sensor device. In this way, a sample can be readily supplied into the liquid sample holding section through the sample supply inlet.

The sucking unit may be manual or automatic and may be, for example, a piston mechanism such as a conventional syringe or dispenser.
The piston of such a piston mechanism may be operated manually or automatically, but automatic operation is preferable since it can reduce the workload of the operator thereof. Automation methods include a method of operating a piston by means of a motor. The motor may be a stepper motor, a DC motor, or the like.

A stepper motor is a motor that rotates by a particular rotation angle per input pulse signal and the rotation angle can be determined by the number of pulses. Thus, it does not need an encoder for positioning. That is, the operation distance of the piston can be controlled based on the number of input pulses.
The rotational motion of the motor is converted to linear motion by using, for example, a gear mechanism and a linear motion mechanism combined with male and female threads, to operate the piston.

The rotational motion of a DC motor is also converted to linear motion by the same method. However, a DC motor needs an encoder that detects the rotational position of the motor in order to control the operation distance of the piston. There is also a linear stepper motor. In the case of this type of motor, the motor contains a linear motion mechanism consisting of combined male and female threads, and a movable bar moves linearly depending on the number of input pulses. Thus, the piston can be directly connected to this bar and the structure is simple.

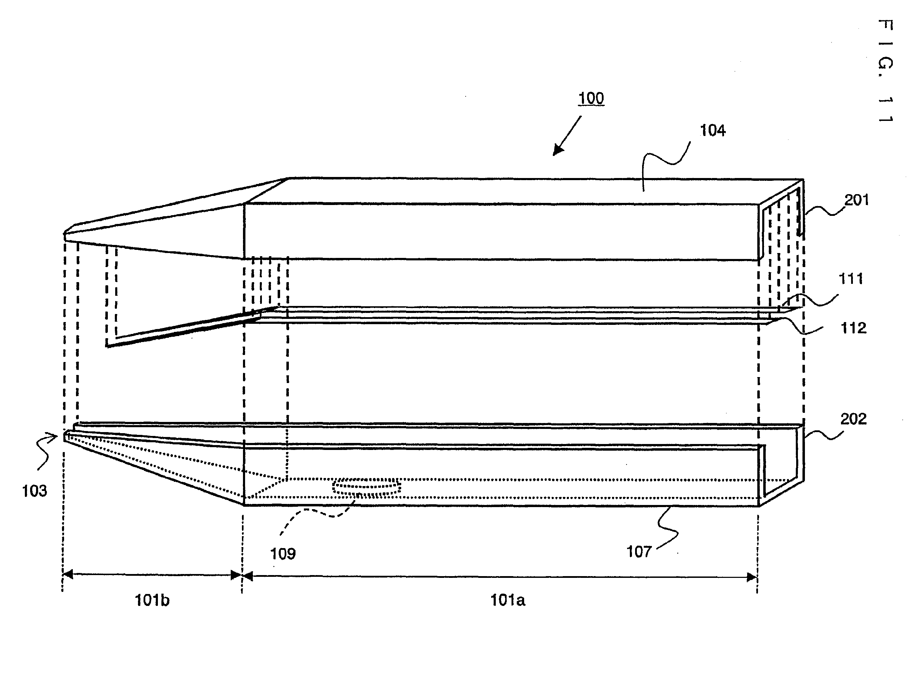

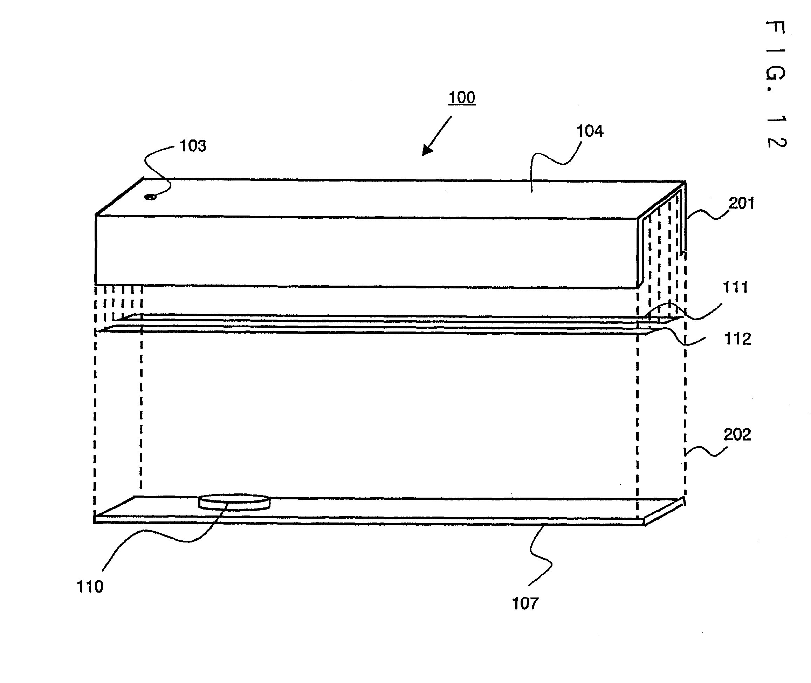

Further, referring to FIGs. 11 and 12, still another modified example is described as an embodiment of the present invention. FIGs. 11 and 12 are exploded perspective views of modified examples of the sensor device of the present invention. The first member 201 has a pair of conductive sections 111 and 112 on the inner surface. In the modified example of FIG. 11, the conductive sections 111 and 112 are mainly provided on the inner wall of the face 104 of the hollow quadrangular prismatic section 101a and extend to near the sample supply inlet 103 at the tip end of the hollow quadrangular pyramidal section 101b. Also, in the modified example of FIG. 12, the sample supply inlet 103 is provided in the face 104 of the first member 201.

These conductive sections 111 and 112 can be formed by covering the inner surface of the first member 201 with an acrylic resin mask having openings of the same shape as the conductive sections 111 and 112, sputtering or depositing gold over the mask, and removing the mask. The same procedure may also be used in deposition instead of sputtering.
The dimensions of the conductive sections 111 and 112 are not particularly limited, and for example, the width may be approximately 2 mm, the length approximately 80 mm, and the thickness approximately 5 µm.
In order to make constant the area of the portions of the conductive sections 111 and 112 to come into contact with a liquid sample irrespective of the amount of liquid sample supplied to the liquid sample holding section, it is preferable to attach an insulating resin film to the conductive sections 111 and 112 such that a part of the conductive sections 111 and 112 is exposed and the remaining part is covered. For example, in FIG. 11, the film is disposed such that the conductive sections 111 and 112 are exposed approximately 10 mm from the end closer to the sample supply inlet 103.

Also, in the above embodiment, the method of forming the conductive sections 111 and 112 by sputtering and deposition has been described; instead, it is also possible to use a method of affixing metal ribbons to the inner surface of the first member 201, a method of printing a metal- or carbon-containing ink on the inner surface of the first member 201, or the like.
In forming the conductive sections, leads and terminals for electrically connecting the conductive sections with the measuring apparatus may be formed simultaneously.

Also, glucose oxidase as an enzyme and ferricyanide ions (potassium salt) as an electron mediator may be carried on the surface of the conductive sections 111 and 112 in this embodiment by the above method. The electron mediator may be potassium ferricyanide, p-benzoquinone, phenazine methosulfate, methylene blue, a ferrocene derivative, a Ru or Os complex, or the like. Also, even when the electron mediator is oxygen, response current can be obtained. Further, they may be used singly or in combination of two or more of them.

In the foregoing embodiments, the example of using sodium hydrogen carbonate as the effervescent agent has been shown, but sodium carbonate may also be used. Also, in the foregoing embodiments, the example of using succinic acid as the organic acid has been shown, but maleic acid, maleic anhydride, phthalic acid, phthalic anhydride, succinic anhydride, citric acid, fumaric acid, or tartaric acid may also be used.
Also, a liquid derived from a living body, such as blood, blood plasma, blood serum, interstitial fluid, urine, or saliva, may also be used as a liquid sample.

### Industrial Applicability

The sensor device according to the present invention is useful as a sensor device for quantifying an analyte in a liquid sample, particularly a biological sample such as blood or urine.

## Claims

1. A sensor device comprising a holding member, said holding member having a liquid sample holding section for holding a liquid sample containing an analyte,
wherein a reagent that reacts or interacts with the analyte in said liquid sample and an effervescent agent that effervesces upon contact with said liquid sample are held at a position of said holding member where said liquid sample and said effervescent agent are to come into contact with said liquid sample introduced into said liquid sample holding section.

2. The sensor device in accordance with claim 1,
wherein said effervescent agent effervesces by producing carbon dioxide.

3. The sensor device in accordance with claim 1 or 2, wherein said effervescent agent comprises at least a hydrogen carbonate or a carbonate.

4. The sensor device in accordance with any one of claims 1 to 3, wherein an organic acid is further held at said position of said holding member.

5. The sensor device in accordance with claim 4,
wherein said organic acid is at least one selected from the group consisting of maleic acid, maleic anhydride, succinic acid, succinic anhydride, phthalic acid, phthalic anhydride, citric acid, fumaric acid, and tartaric acid.

6. The sensor device in accordance with claim 1,
wherein said liquid sample is a liquid derived from a living body.

7. The sensor device in accordance with claim 1,
wherein said reagent is an enzyme or an antibody.

8. The sensor device in accordance with claim 1,
wherein at least a part of an inner face of said liquid sample holding section is hydrophilic.
